Europäisches Patentamt

**European Patent Office**

**Office européen des brevets**

(19)

(11) Publication number: **0 190 781**
**A1**

(12) # EUROPEAN PATENT APPLICATION

(21) Application number: **86200062.7**

(22) Date of filing: **14.01.86**

(51) Int. Cl.⁴: **A 61 L 9/16**

(30) Priority: **17.01.85 NL 8500113**
**14.06.85 NL 8501728**

(43) Date of publication of application:
**13.08.86** Bulletin **86/33**

(84) Designated Contracting States:
**CH DE FR GB IT LI NL**

(71) Applicant: **Vervoort-Kaandorp, Maria Johanna**
**Guurtruda**
**Julianalaan 12**
**NL-6998 AR Laag-Keppel(NL)**

(72) Inventor: **Vervoort-Kaandorp, Maria Johanna Guurtruda**
**Julianalaan 12**
**NL-6998 AR Laag-Keppel(NL)**

(74) Representative: **Cammel, Willem Frans et al,**
**OCTROOIBUREAU ARNOLD & SIEDSMA Sweelinckplein**
**1**
**NL-2517 GK The Hague(NL)**

(54) **Air purifier.**

(57) The invention relates to a device for purifying the air in a more or less enclosed space, for example a toilet.

The invention has for its object to achieve and exceptionally effective purification of the air using very simple means and proposes to this end to provide a device of the type mentioned in the preamble with means for the temporary creation of a localised increase in temperature to above 700°C. Such a temperature increase can for example be achieved with the use of a a spiral filament, if desired in combination with means for forcibly guiding the air to be purified past this spiral filament. Preference will generally be given, however, to a device of the type described which is provided with means for burning fuel. Surprisingly, temporary combustion at a temperature of 1500°C or higher has a very strong purifying effect.

EP 0 190 781 A1

./...

FIG.1

-1-

# Air purifier

The invention relates to a device for purifying the
air in a more or less enclosed space, for example a toilet.

Such a device is known in diverse forms. Use is made
for example of a suction vent, whereby fresh air is drawn in
and mixed with the impure air and the space is gradually
filled via the suction with clean air. Use is also made of
odorant means which, while having no purifying effect on the
air, at least have the effect of influencing the human
olfactory organ such that less nuisance is suffered from
unpleasant odours. It is pointed out further that some of
the propellant gases applied in aerosol sprays are possibly
harmful to the environment.

The use of vents has the drawback that such means are
in themselves comparatively costly and moreover entail
relatively high installation costs, while the use of odorant
means cannot result in any real purification of the air.

The invention has for its object to achieve an
exceptionally effective purification of the air using very
simple means and proposes to this end to provide a device of

the type mentioned in the preamble with means for the temporary creation of a localised increase in temperature to above 700°C. Such a temperature increase can for example be achieved with the use of a spiral filament, if desired in combination with means for forcibly guiding the air to be purified past this spiral filament. Preference will generally be given, however, to a device of the type described which is provided with means for burning fuel. Surprisingly, temporary combustion at a temperature of 1500°C or higher has a very strong purifying effect.

The combustion means can for example comprise a burner connected to a conduit with shut-off valve for liquid or gaseous fuel, and if necessary an ignitor. In a further embodiment the device according to the invention features a carrier for a dosed quantity of solid or liquid fuel and if necessary an ignitor.

The ignitor can for example take the form of an electrical filament fed by a battery or by mains voltage. Use can further be made of an ignitor filled with fuel having a spark ignition on the basis of a scraper wheel with cerium flint, a piezo electric element or an electronic ignition.

As a further refinement a variant can be considered in which the fuel is of a type which emits a pleasant fragrance, for example sandalwood sticks or scented fuels.

The invention will now be explained on basis of the drawing of several arbitrary embodiments. In the drawing:

Fig. 1 shows a partially broken away perspective view of a first embodiment;

Fig. 2 shows an electrical diagram of the embodiment according to fig. 1; and

Fig. 3 shows a second embodiment.

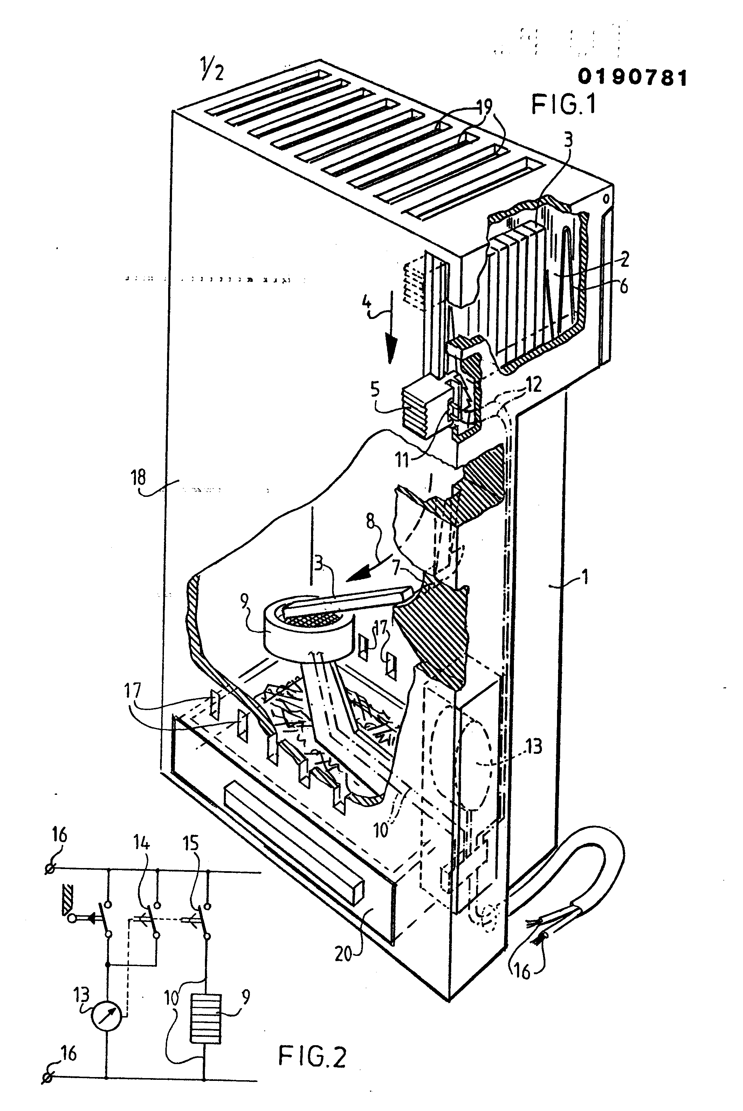

Fig. 1 shows a device 1 for purifying the air in a more or less enclosed space. In this device is present a magazine 2 for dosed quantities of solid fuel in the form of sandal wood sticks 3. By moving a control knob 5 downwards according to arrow 4, the foremost stick, pressed under the influence of a compression spring 6 against the control knob, can in each case be moved downwards such that it comes to rest along a guiding face 7 on a heating element 9 as according to arrow 8 element 9 being supplied via electrical wiring 10.

Control knob 5 bears a copper strip 11, which, in the position of control knob 5 shown in fig. 1 with fully drawn lines, forms a through connection between electrical wiring 12. In this way an electrical control device, schematically represented as a time clock 13 (see fig. 2) can be actuated, whereby, as according to the diagram in fig. 2, a hold contact 14 can be established and a switch 15 accomodated in electrical cable 10 can be closed, such that via cable 10 and switch 15 the supply of current present on electrical connections 18, for example mains current, can set the electrical heating element in operation.

In this manner electrical heating element 9 can be set temporarily in operation to ignite sandalwood stick 3, which thus achieves combustion. Air can enter the housing 18 of the device 1 through holes 17, while the housing is provided on its upper side with outlet openings 19 for combustion products.

A drawer 20 serves as receptacle for the ash from burnt sticks 3. Drawer 20 is removable for emptying.

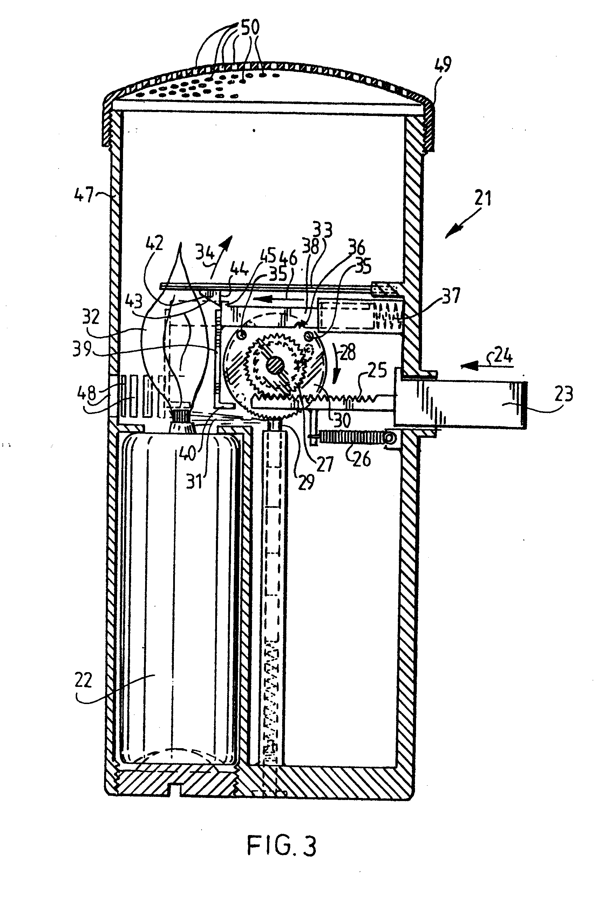

Figure 3 shows a device 21, which in contrast to device 1 according to figures 1 and 2, operates not with solid fuel but with gas present in a holder 22.

A push-button 23 can be pressed in as according to arrow 24, whereby a rack 25 is moved to the left in the drawing counter to the action of a draw spring 26. Rack 25 co-operates with a pinion 27 to move a scraper wheel 30 as according to arrow 28 along a cerium fling 29. In this way there occurs a schematically represented spark 31 which can ignite the flow of gas escaping from gas holder 22.

The flame 32 heats a bimetal 33 which can bend as according to arrow 34.

A number of studs 35 are arranged on scraper wheel 30, which studs co-operate with a recess 36 in a rod 38 which is under load of a compression spring 37, and which bears a lip 39 on its outer end, a bent lower edge 40 of which lip shuts off the gas holder 22 in the position indicated by the broken lines in fig. 3.

By pressing in push-button 23 rod 38 is moved to the right by the movement of projections 35 as according to arrow 28, whereby lower edge 40 is released from the mouth piece 41 of gas holder 22 so that the flame can ignite. Bimetal 33 is then still in its cold, i.e. extended, state. It bears a projection 42 with an inclined rise surface 43 and a locking surface 44 for co-operation with a projection 45 present on the end of rod 38. When push-button 23 is pressed in, the projection 45 can run against and along rise surface 43, thereby pressing projection 42 upward, whereafter it comes to rest against locking surface 44. By heating of bimetal 33 by the now ignited gas flame 32, the bimetal will gradually begin to display a curving, whereby locking face 44 releases projection 45 and rod 38 under the influence of compression spring 37 moves to the left as according to arrow 46 and lower edge 40 against closes off mouth piece 41 of gas holder 22. Temporary combustion of the gaseous fuel is in this way ensured.

The housing 47 of the device bears a number of circumferential openings 48 for supply of air, while the unscrewable cover 49 is provided with outlet openings 50.

Finally, further mention is made of the possibility of using a candle, the wick of which is impregnated with a fragrant substance.

-1-

Claims
------


1. Device for purifying the air in a more or less enclosed space, for example a toilet, <u>characterized by</u> means for the temporary creation of a localised increase in temperature to above 700°C.

2. Device as claimed in claim 1, <u>characterized by</u> means for the combustion of fuel.

3. Device as claimed in claim 2, <u>characterized in that</u> said combustion means comprise a burner connected to a conduit with a closing valve for liquid or gaseous fuel, and if necessary an ignitaor.

4. Device as claim in claim 2, <u>characterized by</u> a carrier for a dosed quantity of solid or liquid fuel, and if necessary an ignitor.

5. Device as claimed in claims 2-4, <u>characterized in that</u> said fuel is of the type that emits a pleasant fragrance when burnt.

0190781

FIG.1

FIG.2

FIG.3

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-2 341 662 (BULTEN-KANTHAL) * Claim 1 * | 1 | A 61 L 9/16 |
| | --- | | |
| A | FR-A-2 482 420 (MIRA LANZA SpA) * Claim 1 * | 2,3 | |
| | --- | | |
| A | FR-A-2 050 493 (DANFOSS) * Page 1, lines 15-18 * | 1 | |
| | --- | | |
| A | US-A-2 818 615 (R.E. BURNESS) | 4 | |
| | --- | | |
| A | FR-A-1 589 603 (DANFOSS) | 1 | |
| | ----- | | |

TECHNICAL FIELDS SEARCHED (Int. Cl.4)

A 61 L

The present search report has been drawn up for all claims

| Place of search | Date of completion of the search | Examiner |
|---|---|---|
| THE HAGUE | 06-05-1986 | PELTRE CHR. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82